# FASCICULE DE BREVET EUROPEEN

(11) **EP 1 064 914 B1**
(45) Date de publication et mention de la délivrance du brevet: **17.05.2006**
(21) Numéro de dépôt: 00401320.7
(22) Date de dépôt: 15.05.2000
(51) Int. Cl.: A61K 8/06, A61K 8/898, A61Q 1/14

(54) **Composition cosmétique de démaquillage et/ou de nettoyage de la peau sous forme d'émulsion eau-dans-huile**
Kosmetische Zusammensetzung zur Schminkproduktenentfernung und/oder zur Hautreinigung in der Form einer Wasser-in-Öl Emulsion
Cosmetic composition for make-up removal and/or skin cleaning as a water-in-oil emulsion

(30) Priorité: 28.06.1999 FR 9908244
(43) Date de publication de la demande: 03.01.2001
(73) Titulaire: L'OREAL, 75008 Paris (FR)
(72) Inventeur: Guillou, Véronique, 92600 Asnières (FR); Carton, Isabelle, 75012 Paris (FR)
(74) Mandataire: Rasson, Catherine

(56) Documents cités:
- EP-A- 0 463 496
- GB-A- 2 136 442
- US-A- 5 871 756
- DATABASE CHEMICAL ABSTRACTS [en ligne] STN; abrégé 130: 342 757, XP002134148 & JP 11 106330 A (AJINOMOTO CO., INC.) 20 avril 1999 (1999-04-20)
- Traduction en Anglais de JP A 11 106 330 (Ajinomoto), demande publiée le 20 Avril 1999
- HAMEYER P.: 'Cyclomethiconhaltige kosmetische W/O-Lotionen auf Basis silicium organischer Emulgatoren' SEIFEN, ÖLE, FETTE no. 10, 1990, pages 392 - 395
- Factsheet: "a guide to Formulating Water-in-Silicone Emulsions with Dow Corning 3225C Formulation Aid" , écrite par K. Kasprzak, 7 pages, non datée, et publiée par Dow Corning
- 'McCutcheon's Emulsifiers and detergents, vol.1', 2000, THE MANUFACTURING CONFECTIONER PUB. CO., USA * pages 42,117, * * page 194 *

## Description

L'invention se rapporte à une composition de démaquillage et/ou de nettoyage se présentant sous forme d'une émulsion eau-dans-huile comportant une forte teneur en eau, au moins une huile démaquillante et un tensioactif siliconé particulier. Cette composition a l'aspect d'une crème et est utilisée notamment pour le démaquillage et/ou le nettoyage de la peau, des lèvres et/ou des yeux.

Les démaquillants et nettoyants du visage se présentant sous forme d'émulsions, classiquement utilisés à ce jour présentent des inconvénients qui ne sont pas les mêmes selon le sens de l'émulsion. Dans le cas des émulsions huile-dans-eau (H/E), la phase aqueuse externe apporte de la fraîcheur. En revanche, la phase huileuse étant la phase interne, elle n'est pas directement disponible pour dissoudre les corps gras et, de ce fait, le démaquillage est moins efficace.

Dans le cas des émulsions eau-dans-huile, très efficaces pour le démaquillage/nettoyage du fait que la phase grasse est directement disponible pour dissoudre les différents corps gras présents sur la peau ou issus du maquillage, leur utilisation est inconfortable du fait de la sensation grasse et lourde apportée par cette phase grasse externe qui subsiste sur la peau.

Or, pour le nettoyage du visage, les femmes recherchent une « sensation d'eau », l'eau étant le symbole de la propreté et de la pureté, valeurs fortement associées au nettoyage, tout en gardant une grande efficacité démaquillante et/ou nettoyante. Les produits nettoyants ou démaquillants utilisant de l'eau sont issus d'une toute autre technologie, à savoir les gels, crèmes ou pains moussants. Pour certains types de peau, notamment, les peaux sèches et sensibles, ces produits conduisent à des tiraillements, des dessèchements voire des intolérances.

Aussi, il subsiste le besoin d'une composition nettoyante et/ou démaquillante efficace qui apporte une sensation de fraîcheur, sans avoir les inconvénients de l'art antérieur.

La demanderesse a maintenant trouvé une composition du type émulsion eau dans huile permettant d'atteindre ces objectifs.

La présente invention a pour objet une composition de nettoyage et/ou de démaquillage, comprenant, dans un milieu physiologiquement acceptable, une phase aqueuse dispersée dans une phase huileuse à l'aide d'un agent émulsionnant siliconé, caractérisée en ce qu'elle comprend (1) au moins 78 % de phase aqueuse, (2) au moins une huile démaquillante choisie parmi les esters d'acide gras comportant au moins 12 atomes de carbone, et (3) comme agent émulsionnant, le diméthicone copolyol de formule (I) : 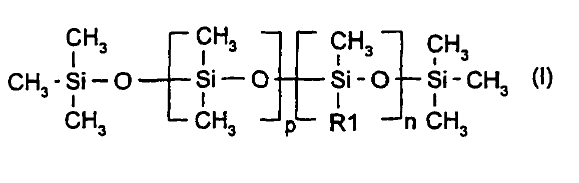 dans laquelle R1 représente -(C₃H₆O)-(C₂H₄O)₁₈-(C₃H₆O)₁₈H, p=394 et n=4, et en ce qu'elle est exempte d'huile hydrocarbonée à chaîne ramifiée.

On entend par « milieu physiologiquement acceptable » un milieu compatible avec la peau, les lèvres, les yeux, le cuir chevelu et/ou les cheveux.

On entend par « exempte d'huile hydrocarbonée à chaîne ramifiée » le fait que la composition ne contienne pas d'huiles hydrocarbonées comportant une chaîne ramifiée, telles que par exemple les isoparaffines, l'isohexadécane et l'isododécane.

En dépit de la quantité importante de phase aqueuse, la composition de l'invention est stable dans le temps. En outre, elle possède une caractéristique rhéologique spécifique qui rend son utilisation dans les domaines considérés, particulièrement intéressante. En effet, lors de l'application sur la peau et après quelques instants de massage, indispensables pour dissoudre les corps gras à éliminer, l'émulsion "casse", c'est-à-dire qu'elle se fluidifie brutalement, libérant ainsi la phase aqueuse et générant une très grande sensation de fraîcheur.

La composition selon l'invention présente de préférence une viscosité allant de 3 Pa.s (30 poises) à 20 Pa.s (200 poises). Cette viscosité est mesurée au Rhéomat 180, c'est-à-dire avec l'appareil RM180 Rheomat de la société METTLER.

La composition selon l'invention comporte au moins 78 % en poids de phase aqueuse par rapport au poids total de la composition et de préférence au moins 80 % du poids total de la composition. La phase aqueuse peut constituer jusqu'à 92 % du poids total de la composition. La phase aqueuse comprend de l'eau et éventuellement des composés hydrosolubles tels que notamment les alcools et les polyols.

L'eau constitue de préférence au moins 65 % et mieux au moins 70 % du poids total de la composition.

Par ailleurs, selon un mode préféré de réalisation de l'invention, la phase aqueuse contient comme composés hydrosolubles, un ou plusieurs alcools inférieurs ou polyols. On entend par alcool inférieur les alcools comportant de 1 à 6 et plus particulièrement de 1 à 4 atomes de carbone, tels que l'éthanol. Comme polyols, on peut citer par exemple la glycérine, le propylène glycol et les polyéthylène glycols (PEG-8 par exemple). Ces alcools et/ou polyols peuvent être présents dans la composition en une quantité allant de préférence de 0,5 à 25 % et de 1 à 15 % du poids total de la composition.

Par ailleurs, la composition de l'invention contient comme agent émulsionnant et de préférence comme seul agent émulsionnant, le diméthicone copolyol de formule (I). Ce diméthicone copolyol peut se présenter sous forme d'un mélange avec une huile de silicone volatile ou non volatile, et notamment avec les cyclométhicones (D4 ou D5) et/ou les polydiméthylsiloxanes de différentes viscosités et notamment 5 cst et 10 cst.

On peut utiliser notamment dans la composition selon l'invention les mélanges suivants commercialisés par la société Dow Corning :
- mélange de composé de formule (I), de tétracyclométhicone (D4) et d'eau (rapport pondéral 10/88/2), commercialisé sous la dénomination DC 3225C ;
- mélange de composé de formule (I), de pentacyclométhicone (D5) et d'eau (rapport pondéral 10/88/2), commercialisé sous la dénomination DC 5225C ;
- mélange de composé de formule (I) et de polydiméthylsiloxane 5 cst (rapport pondéral 10/90), commercialisé sous la dénomination DC 3225C in 200 Fluid 5 cst ;
- mélange de composé de formule (I) et de polydiméthylsiloxane 10 cst (rapport pondéral 10/90), commercialisé selon sous la dénomination DC 3225C in 200 Fluid 10 cst ;
- mélange de composé de formule (I) et de pentacyclométhicone (D5) (rapport pondéral 43/57), commercialisé sous la dénomination DC 5185C.

L'agent émulsionnant de formule (I) est présent de préférence en une quantité en matière active allant de 0,1 à 5 % et mieux de 0,5 à 3 % en poids par rapport au poids total de la composition.

Même quand la composition est exempte de tout autre agent émulsionnant, elle reste stable dans le temps.

De préférence, le rapport pondéral phase huileuse/agent émulsionnant est égal ou supérieur à 5 et mieux égal ou supérieur à 8.

La phase huileuse de la composition selon l'invention contient au moins une huile démaquillante choisie parmi les esters d'acide gras comportant au moins 12 atomes de carbone.

L'huile démaquillante est choisie parmi les esters d'acide gras comportant au moins 12 atomes de carbone. Ces esters sont de préférence obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou di-esters.

De manière préférée, l'huile démaquillante est choisie dans le groupe des esters qui ne comportent aucune insaturation et/ou aucun groupement éther ou hydroxyle. De façon encore plus avantageuse, l'huile démaquillante est un ester saturé qui ne renferme aucun groupement éther ni hydroxyle.

Ainsi, l'huile démaquillante de la composition conforme à l'invention peut être notamment choisie dans le groupe comprenant le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.

La quantité d'huile(s) démaquillante(s) peut constituer tout ou une partie de la phase huileuse, et par exemple de 1 à 90 % et mieux de 5 à 70 % du poids total de la phase huileuse.

La phase huileuse peut contenir en outre tous les corps gras et notamment les huiles non démaquillantes autres que celles indiquées ci-dessus, classiquement utilisés dans le domaine cosmétique. Comme autres huiles susceptibles d'être présentes dans la phase huileuse, on peut citer par exemple les huiles minérales à chaîne hydrocarbonée linéaire, telles que l'huile de vaseline ; les huiles d'origine végétale comme l'huile de noyaux d'abricot ; les huiles de synthèse ; les huiles de silicone volatiles ou non volatiles et les huiles fluorées. Les autres corps gras susceptibles d'être présents dans la phase huileuse peuvent être par exemple les acides gras, les alcools gras et les cires.

La phase huileuse est présente dans la composition selon l'invention en une quantité allant de 1 à 21,5 % et de préférence de 3 à 18 % en poids par rapport au poids total de la composition.

De façon connue, la composition de l'invention peut en outre contenir des adjuvants habituels dans le domaine cosmétique, tels que des actifs hydrophiles ou lipophiles, des conservateurs, des antioxydants, des parfums, des solvants, des charges, des filtres, des matières colorantes (piments ou colorants), des agents basiques (triéthanolamine) ou acides et encore des vésicules lipidiques. Ces adjuvants sont utilisés dans les proportions habituelles dans le domaine cosmétique ou dermatologique, et par exemple de 0,01 à 30 % du poids total de la composition, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour la composition.

La présente invention a encore pour objet un procédé cosmétique de démaquillage et/ou de nettoyage de la peau, des lèvres et/ou des yeux, caractérisé par le fait que l'on applique sur la peau, les lèvres et/ou les yeux, une composition telle que définie ci-dessus.

L'invention a aussi pour objet l'utilisation cosmétique de la composition telle que définie ci-dessus pour le démaquillage et/ou le nettoyage de la peau, des lèvres et/ou des yeux.

L'exemple ci-après de compositions selon l'invention est donné à titre d'illustration et sans caractère limitatif. Les quantités y sont données en % en poids, sauf mention contraire.

### Exemple : Emulsion pour le démaquillage du visage

### A. Phase huileuse

- Diméthicone copolyol de formule (I) dans pentacyclométhicone et eau (1018812) (DC 5225 C) 9,2 %
- Huile de vaseline 3,2 %
- Palmitate d'isopropyle 3 %

### B. Phase aqueuse

- Chlorure de sodium 2,5 %
- Glycérine 5 %
- Ethanol 5 %
- Conservateurs 0,65 %
- Sel de sodium d'EDTA 0,1 %
- PEG-8 4 %
- Eau qsp 100 %

Mode opératoire : On prépare la phase A par mélange des constituants sous agitation à 600 tours/minute. On prépare par ailleurs la phase B et on introduit une partie (environ 1/10) de la phase B dans la phase A très lentement sous agitation. On ajoute ensuite le reste de la phase B plus rapidement toujours sous agitation, et on maintient l'agitation pendant un certain temps.

On obtient une crème blanche ayant une viscosité mesurée au RHEOMAT 180, mobile 4, de 5,74 Pa.s (57,4 poises) au temps zéro. Cette viscosité se stabilise après 10 minutes à 4,56 Pa.s (45,6 poises).

Lors du démaquillage ou nettoyage, la composition est massée sur la peau pendant quelques secondes (5 à 10 secondes). Au bout de ce temps, elle « casse » apportant une grande sensation de fraîcheur tout en démaquillant ou nettoyant parfaitement la peau. La crème est ensuite essuyée avec un coton ou un tissu.

## Revendications

1. Composition de nettoyage et/ou de démaquillage, comprenant, dans un milieu physiologiquement acceptable, une phase aqueuse dispersée dans une phase huileuse à l'aide d'un agent émulsionnant siliconé, **caractérisée en ce qu'**elle comprend (1) au moins 78 % de phase aqueuse, (2) au moins une huile démaquillante choisie parmi les esters d'acide gras comportant au moins 12 atomes de carbone, et (3) comme agent émulsionnant, le diméthicone copolyol de formule (I) : 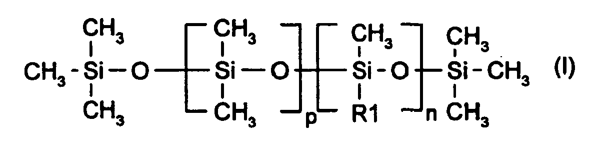 dans laquelle R1 représente -(C₃H₆O)-(C₂H₄O)₁₈-(C₃H₆O)₁₈H, p=394 et n=4, et **en ce qu'**elle est exempte d'huile hydrocarbonée à chaîne ramifiée.

2. Composition selon la revendication 1, **caractérisée par le fait que** l'agent émulsionnant est en mélange avec au moins une huile de silicone.

3. Composition selon la revendication 1 ou 2, **caractérisée par le fait que** l'agent émulsionnant est présent en une quantité allant de 0,5 à 5 % en poids par rapport au poids total de la composition.

4. Composition selon l'une quelconque des revendications précédentes, **caractérisée par le fait que** la phase huileuse est présente en une quantité allant de 1 à 21,5 % en poids par rapport au poids total de la composition.

5. Composition selon l'une quelconque des revendications précédentes, **caractérisée par le fait que** le rapport pondéral phase huileuse/agent émulsionnant est égal ou supérieur à 5.

6. Composition selon l'une quelconque des revendications précédentes, **caractérisée par le fait que** l'huile démaquillante est choisie parmi les esters obtenus à partir d'un alcool à chaîne droite ou ramifiée, comportant de 1 à 17 atomes de carbone et d'un acide gras à chaîne droite ou ramifiée, comportant de 14 à 22 atomes de carbone.

7. Composition selon la revendication précédente, **caractérisée par le fait que** l'huile démaquillante est choisie dans le groupe comprenant le palmitate d'éthyl-2 hexyle, le myristate d'éthyl-2 hexyle, le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle et leurs mélanges.

8. Composition selon l'une quelconque des revendications précédentes, **caractérisée par le fait que** la phase aqueuse contient au moins un alcool comportant de 1 à 6 atomes de carbone et/ou un polyol.

9. Composition selon la revendication précédente, **caractérisée par le fait que** la quantité d'alcool comportant de 1 à 6 atomes de carbone et/ou de polyol va de 0,5 à 25 % en poids par rapport au poids total de la composition.

10. Utilisation cosmétique de la composition selon l'une quelconque des revendications précédentes pour le démaquillage et/ou le nettoyage de la peau, des lèvres et/ou des yeux.

11. Procédé cosmétique de démaquillage et/ou de nettoyage de la peau, des lèvres et/ou des yeux, **caractérisé en ce que** l'on applique sur la peau, les lèvres et/ou les yeux, une composition selon l'une quelconque des revendications 1 à 9.

## Claims

1. Cleansing and/or make-up-removing composition comprising, in a physiologically acceptable medium, an aqueous phase dispersed in an oily phase with the aid of a silicone emulsifier,
**characterized in that** it comprises (1) at least 78% of aqueous phase, (2) at least one make-up-removing oil chosen from fatty acid esters containing at least 12 carbon atoms, and (3), as emulsifier, the dimethicone copolyol of formula (I) : 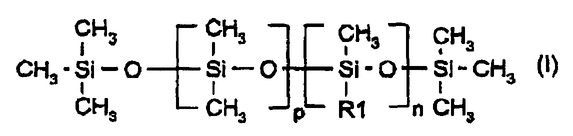 in which R1 represents -(C₃H₆O)-(C₂H₄O)₁₈-(C₃H₆O)₁₈H, p = 394 and n = 4,
and **in that** it is free of branched-chain hydrocarbon-based oil.

2. Composition according to Claim 1, **characterized in that** the emulsifier is in a mixture with at least one silicone oil.

3. Composition according to Claim 1 or 2, **characterized in that** the emulsifier is present in an amount ranging from 0.5 to 5% by weight relative to the total weight of the composition.

4. Composition according to any one of the preceding claims, **characterized in that** the oily phase is present in an amount ranging from 1 to 21.5% by weight relative to the total weight of the composition.

5. Composition according to any one of the preceding claims, **characterized in that** the oily phase/emulsifier weight ratio is greater than or equal to 5.

6. Composition according to any one of the preceding claims, **characterized in that** the make-up-removing oil is chosen from esters obtained from a straight-chain or branched-chain alcohol containing from 1 to 17 carbon atoms and from a straight-chain or branched-chain fatty acid containing from 14 to 22 carbon atoms.

7. Composition according to the preceding claim, **characterized in that** the make-up-removing oil is chosen from the group comprising 2-ethylhexyl palmitate, 2-ethylhexyl myristate, isopropyl palmitate, isopropyl myristate, diisopropyl adipate, dioctyl adipate, 2-ethylhexyl hexanoate, ethyl laurate, methyl myristate, octyldodecyl octanoate, isodecyl neopentanoate, ethyl myristate, myristyl propionate, 2-ethylhexyl 2-ethylhexanoate, 2-ethylhexyl octanoate, 2-ethylhexyl caprate/caprylate, methyl palmitate, butyl myristate, isobutyl myristate, ethyl palmitate, isohexyl laurate, hexyl laurate and isopropyl isostearate, and mixtures thereof.

8. Composition according to any one of the preceding claims, **characterized in that** the aqueous phase contains at least one alcohol containing from 1 to 6 carbon atoms and/or one polyol.

9. Composition according to the preceding claim, **characterized in that** the amount of alcohol containing from 1 to 6 carbon atoms and/or of polyol ranges from 0.5 to 25% by weight relative to the total weight of the composition.

10. Cosmetic use of the composition according to any one of the preceding claims, for removing make-up from and/or for cleansing the skin, the lips and/or the eyes.

11. Cosmetic process for removing make-up from and/or for cleansing the skin, the lips and/or the eyes, **characterized in that** a composition according to any one of Claims 1 to 9 is applied to the skin, the lips and/or the eyes.

## Patentansprüche

1. Zusammensetzung zur Reinigung und/oder zum Abschminken, die in einem physiologisch akzeptablen Medium eine wässrige Phase enthält, die mit einem siliconierten Emulgator in einer Ölphase dispergiert ist, **dadurch gekennzeichnet, dass** sie (1) mindestens 78 % wässrige Phase, (2) mindestens ein Abschminköl, das unter den Fettsäureestern mit mindestens 12 Kohlenstoffatomen ausgewählt ist, und (3) als Emulgator das Dimethiconcopolyol der Formel (I) enthält: 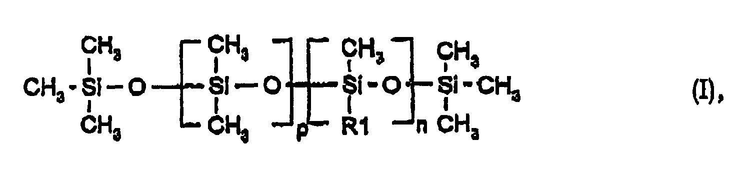 worin R₁ -(C₃H₆O)-(C₂H₄O)₁₈-(C₃H₆O)₁₈H bedeutet, p = 394 und n = 4, und **dadurch**, dass sie kein Kohlenwasserstofföl mit verzweigter Kette enthält.

2. Zusammensetzung nach Anspruch 1, **dadurch gekennzeichnet, dass** der Emulgator im Gemisch mit mindestens einem Siliconöl vorliegt.

3. Zusammensetzung nach Anspruch 1 oder 2, **dadurch gekennzeichnet, dass** der Emulgator in einer Menge von 0,5 bis 5 Gew.-%, bezogen auf das Gesamtgewicht der Zusammensetzung, vorliegt.

4. Zusammensetzung nach einem der vorhergehenden Ansprüche, **dadurch gekennzeichnet, dass** die Ölphase in einer Menge von 1 bis 21,5 Gew.-%, bezogen auf das Gesamtgewicht der Zusammensetzung, enthalten ist.

5. Zusammensetzung nach einem der vorhergehenden Ansprüche, **dadurch gekennzeichnet, dass** das Gewichtsverhältnis Ölphase/Emulgator 5 beträgt oder darüber liegt.

6. Zusammensetzung nach einem der vorhergehenden Ansprüche, **dadurch gekennzeichnet, dass** das Abschminköl unter den Estern ausgewählt ist, die ausgehend von einem Alkohol mit gerader oder verzweigter Kette, der 1 bis 17 Kohlenstoffatomen aufweist, und einer Fettsäure mit gerader oder verzweigter Kette erhalten werden, die 14 bis 22 Kohlenstoffatome enthält.

7. Zusammensetzung nach dem vorhergehenden Anspruch, **dadurch gekennzeichnet, dass** das Abschminköl unter 2-Ethylhexylpalmitat, 2-Ethylhexylmyristat, Isopropylpalmitat, Isopropylmyristat, Diisopropyladipat, Dioctyladipat, 2-Ethylhexylhexanoat, Ethyllaurat, Methylmyristat, Octyldodecyloctanoat, Isodecylneopentanoat, Ethylmyristat, Myristylpropionat, 2-Ethylhexyl-2-ethylhexanoat, 2-Ethylhexyloctanoat, 2-Ethylhexylcaprat/caprylat, Methylpalmitat, Butylmyristat, Isobutylmyristat, Ethylpalmitat, Isohexyllaurat, Hexyllaurat, Isopropylisostearat und deren Gemischen ausgewählt ist.

8. Zusammensetzung nach einem der vorhergehenden Ansprüche, **dadurch gekennzeichnet, dass** die wässrige Phase mindestens einen Alkohol mit 1 bis 6 Kohlenstoffatomen und/ oder ein Polyol enthält.

9. Zusammensetzung nach dem vorhergehenden Anspruch, **dadurch gekennzeichnet, dass** die Menge des Alkohols mit 1 bis 6 Kohlenstoffatomen und/oder des Polyols im Bereich von 0,5 bis 25 Gew.%, bezogen auf das Gesamtgewicht der Zusammensetzung, liegt.

10. Kosmetische Verwendung der Zusammensetzung nach einem der vorhergehenden Ansprüche zum Abschminken und/oder zur Reinigung der Haut, der Lippen und/oder der Augen.

11. Kosmetisches Verfahren zum Abschminken und/oder zur Reinigung der Haut, der Lippen und/ oder der Augen, **dadurch gekennzeichnet, dass** auf die Haut, die Lippen und/ oder die Augen eine Zusammensetzung nach einem der Ansprüche 1 bis 9 aufgetragen wird.
